(19) Europäisches Patentamt
European Patent Office
Office européen des brevets

(11) **EP 2 375 248 A1**

(12) **EUROPEAN PATENT APPLICATION**

(43) Date of publication:
**12.10.2011 Bulletin 2011/41**

(51) Int Cl.:
*G01N 29/02* (2006.01)   *A61B 5/00* (2006.01)

(21) Application number: **11397506.4**

(22) Date of filing: **11.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.04.2010 FI 20100145**

(71) Applicant: **Delfin Technologies Oy**
**70211 Kuopio (FI)**

(72) Inventors:
• **Lahtinen, Tapani**
**70260, Kuopio (FI)**
• **Nuutinen, Jouni**
**70420, Kuopio (FI)**

(74) Representative: **Rahkonen, Erkki Juhani**
**Tampereen Patenttitoimisto Oy**
**Hermiankatu 1 B**
**33720 Tampere (FI)**

(54) **Measurement of matter accumulated on the surface of skin or in hair**

(57)    The invention relates to a method for measuring matters accumulated onto the surface of the skin or in hair, in which method a measuring sensor (3) is placed in contact with the surface of the skin or hair, wherein matter from the surface of the skin or hair adheres to the surface of the measuring sensor (3). The mass of the matter adhered to the measuring sensor (3) is weighed by determining the specific frequencies of the loaded measuring sensor (3) and the clean measuring sensor (3) and, by means of these, the change in the specific frequency of the measuring sensor (3) and in the mass, which is the mass of the matter adhered to the measuring sensor (3), In the method according to the invention, the specific frequency of the measuring sensor (3) is determined by generating a frequency scanning signal on both sides of the specific frequency of the measuring sensor (3) and by guiding the frequency scanning signal through the measuring sensor (3). The response of the frequency scanning signal in the measuring sensor (3) is measured and analyzed, thereby determining the specific frequency of the measuring sensor.

Fig. 3

## Description

Field of the invention

The invention relates to a method according to the preamble of claim 1.

Background of the invention

Skin is an organ made up of layers, the surface layer formed by epidermis which is rich in cells. The surface layer of the epidermis, *stratum corneum,* is constantly exfoliating, and typically, the epidermis is renewed in about two weeks. Beneath the epidermis, there is the dermis whose circulation is taken care of by the superficial and deep vascular plexus. Further, beneath the epidermis, there is subcutaneous fat. Organs associated with the skin include hair, nails, as well as sweat glands and sebaceous glands.

The sebaceous glands in the skin secrete sebum onto the surface of the skin. Sebum is an oily mixture of lipids, and it also contains, among other things, keratin, fatty acids, cholesterol, as well as structural elements of cutaneous cells. The function of the sebum is to protect and lubricate the skin; if the secretion of sebum is insufficient, the skin becomes dry. On the other hand, if the secretion is too abundant, the surface of the skin becomes oily (oily skin). The secretion of sebum varies according to anatomical location, sex, age, skin temperature, and time of day. In general, the secretion of sebum is more abundant in men than in women, and for both sexes the secretion is most abundant at the age of approximately 30 to 40. In women, the phase of the menstrual cycle also affects the quantity of sebum secretion. Of skin areas, sebaceous glands are found particularly in the areas of the scalp, the forehead, the face, as well as the chest and the back. In other words, sebum is secreted in those skin locations where inflammation of the sebaceous glands, or acne, occurs. According to reference publications, the secretion of sebum on the forehead is about fivefold compared with the area of the back.

The function of the sebaceous glands is assessed by determining the sebum excretion rate, whose unit is micrograms per $cm^2$ and which can also be given in a unit of time, that is, in micrograms per $cm^2$ in an hour. Typically, when the quantity of sebum secretion is tested, the sebum is first wiped off the skin, for example with a cleanser containing alcohol. After that, the secretion of sebum onto the skin is monitored for a few hours. According to the prior art, the secretion of sebum is most abundant during the first about 1 to 3 hours from the wiping, after which the secretion rate reaches a plateau stage.

Also other matter than sebum is accumulated onto the skin. Sweat glands secrete salty water, or sweat, onto the skin. The secretion of sweat is closely related to temperature control. The skin is also permeable to water, and its quantity is given in transepidermal water loss (TEWL). Measuring it is known from the method according to US patent 6,966,877. The renewal of skin involves the property that the surface layer of the skin (the stratum corneum) is constantly exfoliating. The surface layer of the skin functions as a barrier to water, and this skin function is called the barrier function. Sometimes it is desirable to increase the permeability of the skin to water by making the exfoliating surface layer thinner, wherein an opening that is more permeable to water is formed in the surface layer of the skin. Typically, the stratum corneum is made thinner by removing the surface layer by an adhesive contact; typically, such a contact is provided by applying a tape, and the method is called tape stripping. To condition the thinning of the stratum corneum and thereby the increased permeability to water, it is necessary to assess the quantity of stratum corneum removed. The water barrier of the skin can be changed by applying creams onto the skin. Thus, the water permeability is reduced; in other words, the cream is used to prevent the loss of water from the skin. Creams applied onto the skin are absorbed into the skin, a part being sometimes left on the surface of the skin.

The secretion of sebum has been measured gravimetrically by absorbing sebum into a porous cloth or paper and weighing the sample with sensitive scales. In some cases, the sebum has first been dissolved in ether and the sample has been weighed after the ether has been evaporated. Optical methods are in use as well. In patent publication US 4,224,950, the transparency of frosted glass applied onto the skin is changed by sebum accumulated onto it. The change in the transparency of the skin causes a need to focus the image produced by the device, after which the measurement result can be read on the scale of the device. The methods known from the patent document US 5,935,521 and US 5,119,828 can also be used to indirectly measure the secretion of sebum. In the methods, a porous and opaque plastic film is applied onto the skin, the pores in skin contact being filled with sebum. According to the reference documents, the duration of the skin contact is about 30 seconds. In the method according to US 5,935,521, the plastic film has a pore density of 20 to 50% of the surface area, and the size of the pores is 0.03 to 0.15 micrometers. The thickness of the film is 20 to 30 micrometers. Applied onto the skin and in contact with sebum, such a film becomes transparent. This change in the optical light transmission of the film is measured, and its magnitude is an indirect measure of the quantity of the sebum secretion. The methods have the disadvantage that they require optical focusing. In the method and the respective apparatus, arbitrary units (AU) are typically used, and the result is not given in the SI units of micrograms per $cm^2$. Consequently, the method according to US patent 5,935,521 relates to an indirect measurement of sebum quantity.

Document US 3,863,495 discloses a method for determining the mass of a substance by means of a quartz crystal. The mass accumulated onto the crystal changes the oscillation frequency of the crystal, and this change is, in turn, proportional to the quantity of the mass on the surface of the crystal. In the method, the change in the frequency of the quartz crystal is measured, and its magnitude is used for calculating the mass by means of the known relation.

Brief summary of the invention

It is an aim of the present invention to introduce a method for eliminating the above-described drawbacks in the measurement of sebum and other matter secreted, adhered or dispensed onto the skin or hair. In particular, the aim of the invention is to introduce a method for measuring the quantity of sebum, sweat, scurf, and other secretion accumulated or secreted onto the surface of skin or hair, as well as cream dispensed or dirt or other impurities fallen onto the surface of the skin. These matters to be measured are either secreted naturally onto the skin or they have fallen onto the skin, adhered by contact, or dispensed on purpose. All the measurements can be made either from the skin or from the surface of hair. The quantity of the matter to be measured is assessed by weighing. Furthermore, it is an aim of the invention to introduce an advantageous and quick method for measuring the quantity of sebum secretion on the skin or hair, or creams and other substances dispensed onto the skin, in a painless manner directly from the surface of the skin or from hair. The method does not set limits on the location of measuring nor affect the skin itself in any way.

In the method according to the invention, a measuring sensor is applied onto the surface of the skin or against hair, wherein secretion, sebum, water, scurf, dirt, impurities, or remains of a product applied onto the surface of the skin or hair will adhere to it from the surface of the skin or from hair. The basic idea in the method according to the invention is that the mass of the matter adhered onto the surface of the measuring sensor is weighed by determining the specific frequencies of the loaded measuring sensor and the unloaded measuring sensor in a new way that is inventive with respect to the prior art. This is done by determining the changed specific frequency of the measuring sensor by performing a frequency scan on both sides of the known resonance frequency of the measuring sensor, which scan is directed through the measuring sensor. Signals are measured across a resistive load coupled in series in front of the measuring sensor, which signals are filtered by low pass filters and amplified. The signals are analyzed, and the specific frequencies of the loaded and clean measuring sensor are determined from them. By comparing these specific frequencies, it is possible to find out the change in the specific frequency and thereby the respective change in the mass of the measuring sensor, that is, the mass of the matter adhered to the sensor. More precisely, the method according to the invention is characterized in what will be presented in the characterizing part of claim 1.

In the method according to the invention, for example a quartz crystal is advantageously used as the measuring sensor. The mass of the matter adhered onto the surface of such a measuring sensor can be calculated from the specific frequencies determined in the above-mentioned way by the so-called Sauerbrey equation:

$$\Delta f = (-2 f_0^2\, \Delta_m) / (A (\rho_q\, \mu_q)^{1/2}),$$

in which $f_0$ is the specific frequency of the crystal, $\Delta_m$ is the mass placed onto the surface of the crystal, and A is the surface area of the piezoelectronically active area of the crystal. Furthermore, $\mu_q$ is the module of rigidity of the crystal, and $\rho_q$ is the density of the crystal.

Significant advantages are achieved by means of the invention. The measurement is quick, taking only a few seconds, and the measurement by the device based on the invention does not interfere with the normal physiology of the skin in any way.

By means of the invention, it is possible to assess the quantity of sebum excretion, the absorption of drugs applied onto the skin, or the permeability of the skin to water, that is, the barrier function.

In an advantageous embodiment of the invention, the measurement is taken freehand in only a few seconds. In this way, the local quantity of sebum can be determined quickly, and the special rate of sebum secretion can be determined by repeated measurements at different moments of time.

In a further advantageous embodiment of the invention, the device is used for measuring not only sebum but also the absorption of a product dispensed onto the skin or the remains of a product on the skin. The quantity of water secreted through the skin or evaporated from the surface of the skin can also be measured. Even damage caused in the skin barrier to water, *i.e.* the barrier function, can be assessed by this same method by weighing the quantity of the surface layer (stratum corneum) to be removed from the skin. Impurities and contaminants fallen onto or otherwise adhered to the skin, for example by contact, can also be weighed by the method presented in the invention.

It should be noted that in this application, a loaded measuring sensor refers to a measuring sensor, onto which some matter has adhered from the surface of the skin or hair, for example dirt accumulated onto the surface of the skin or hair, or sebum, sweat, scurf, or another secretion from the skin or hair. A clean measuring sensor refers to a measuring sensor, on which there is no such matter, or which measuring sensor has been cleaned substantially free from this matter.

Description of the drawings

In the following, the method according to the invention and the respective apparatus will be described in more detail with reference to the appended drawings, in which

Fig. 1 shows a measuring sensor, on which matter to be measured has been accumulated,
Fig. 2 shows a principle view of the structure of the measuring head shown in

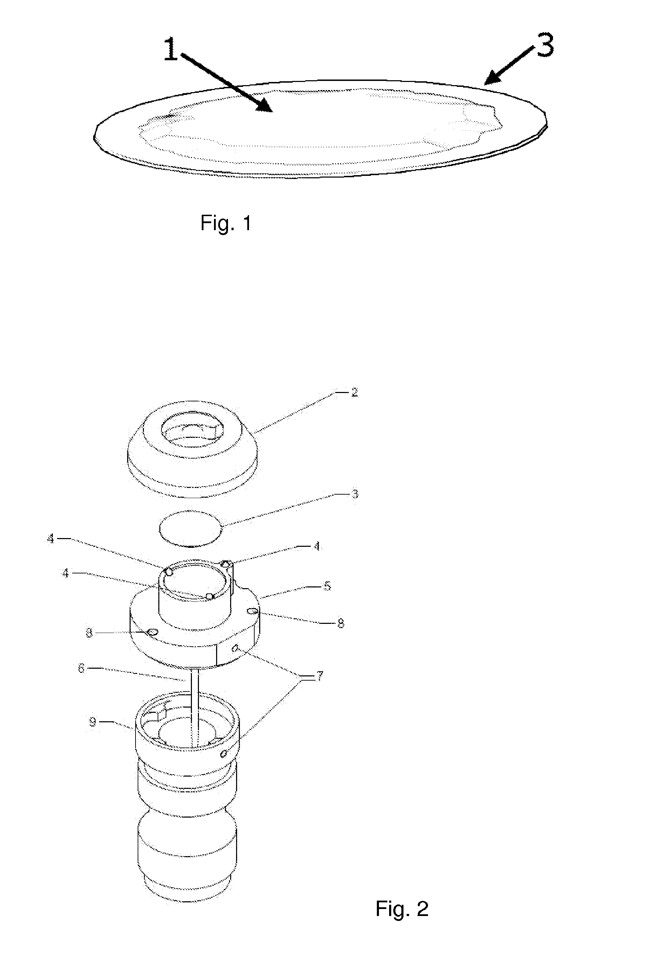

Fig. 1, and

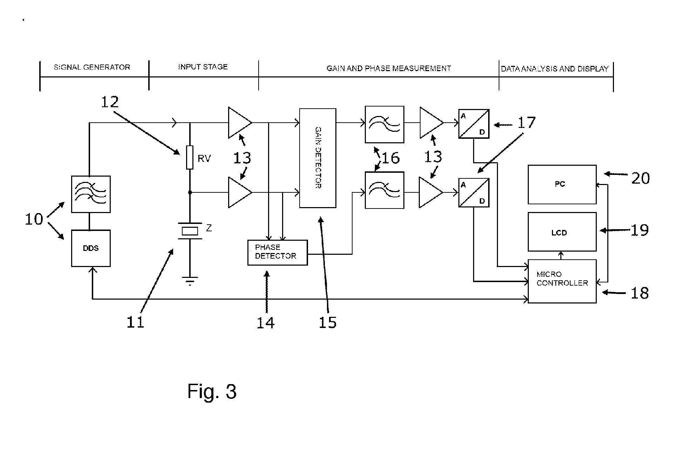

Fig. 3 shows a block chart of a device applying the measuring sensor shown in Figs. 1 and 2.

Detailed description of the invention

Figure 1 shows a replaceable measuring sensor 3, on which there is a mass 1 to be weighed.

A measuring head shown in Fig. 2 comprises a holder 2 for the measuring head, a replaceable measuring sensor 3, contact pins for the measuring sensor, a positioner 5 for the contact pins, magnets 8 for locking the holder 2 for the measuring sensor, perforations 7 for locking the positioner, a coaxial cable 6, and the body 9 of the measuring head. The coaxial cable 6 is connected to the contact pins 4 inside the positioner 5.

In Fig. 3, the block chart of a measuring device comprises a sine wave signal generator 10 formed by an oscillator and a band pass filter, a connector 11 for a measuring sensor, a serial resistance 12, signal amplifiers 13, a phase detector 14, a gain detector 15, low pass filters 16, A/D converters 17, a micro controller 18 for controlling the oscillator and analyzing the data, an LCD for displaying 19 the results, and a PC 20 for collecting data.

When using the measuring device according to the method of the invention, shown in Figs. 1 to 3, the measuring sensor 3 is placed in contact with the surface of the skin. Thus, secretion from the surface of the skin adheres to the surface of the measuring sensor 3, which secretion may be sebum, water, exfoliating part of the surface layer of the skin, or other matter mentioned above and secreted onto the surface of the skin. Furthermore, remains of cream or drug applied onto the skin, or dirt, impurity, dust, or contamination adhered to the skin, may also adhere to the surface of the measuring sensor 3.

Upon measuring the resonance frequency of the measuring sensor both when clean and when loaded (for determining the mass of the measuring sensor), the device functions in such a way that the micro controller 18 controls the signal generator 10 to perform a frequency scan on both sides of the known resonance frequency of the clean measuring sensor. This signal is led through the measuring sensor 3 via the serial resistance 12 and the connector 11 for the measuring sensor. The second end of the connector for the measuring sensor is grounded. The signal is led from both sides of the serial resistance to the signal amplifiers 13, after which the phase difference between the signals is converted to a direct voltage by the phase detector 14, and the amplitude of the signal is converted to a direct voltage by the gain detector 15. Both signals are filtered by the low pass filters 16, after which the signals are led to the amplifiers 13 before the A/D converters 17. The micro controller 18 stores the results of the A/D conversion and analyzes the signals by determining the momentary resonance frequency of the measuring sensor. The measurement result on the matter adhered in contact to the measuring sensor is obtained by computing the difference between the resonance frequencies of the measuring sensor when unloaded and loaded, which difference is directly proportional to the quantity of the mass. The measurement result can be displayed on the display 19 of the device and transmitted, if necessary, to the PC 20 for collecting data.

The measuring arrangement shown in Fig. 3 is only one example implementation of the method by a technique known as such.

In some measuring devices according to the method of the invention, the surface of the measuring sensor can be provided with a surface treatment in such a way that it will be sensitive to the matter to be measured, or to a component of it. The surface treatment used may be coating or texturing. By selecting a suitable coating substance or texturing method for the surface of the measuring sensor, its function can be affected by at least the following different ways:

1) The coating substance either binds or adheres like a tape to the matter to be measured as such but in a more specific way, so that the quantity of the matter to be measured may be controlled better than on a conventional tape. In this approach, the thickness of the coating material may be significantly thinner than a conventional tape film, for example between 10 nm and 1 $\mu$m. As a result, it is possible to attach a greater mass onto the measuring sensor itself, without the load caused by the weight of a tape. There are more material alternatives than for tapes.

2) The surface of the measuring sensor may be provided with a pattern of pores or, for example, apertures, wherein it is also possible to control the quantity or location of the matter to be measured, for example sebum, on the measuring sensor.

3) The surface of the measuring sensor may be provided with such a pattern that the matter to be measured will only adhere to desired locations. This allows e.g. smaller quantities of sebum without changing the size of the sensor itself. The pattern may consist of e.g. hydrophobic and/or hydrophilic polymers. The surface of the measuring sensor can be provided with a pattern in the form of an optical grid on the area of adherence to the matter to be measured, e.g. sebum, wherein optical reflection can be used to determine properties of the matter to be measured, for example the thickness of its layer.

4) The material to be coated may be a polymer that absorbs sebum or other fat, or a porous membrane which enables the collection of sebum.

There are several suitable methods for providing the measuring sensor with a coating and a pattern: For example the so-called spin coating method, which is applied for example in the production of electronics (in the application of photosensitive resist) onto a silicon or glass wafer. The method can also be used to make a very small layer thickness in a controlled manner, down to 5 to 10 nanometres. Another possible method is spray coating. This method is also a well-known method for producing suitable film thicknesses. Any possible printing methods, which are known as such, are also included in the scope of the embodiment, and they can be presented in the form of a list without excluding any methods not mentioned in the list: ink jet, rotogravure, flexography, serigraphy, tampo printing, offset printing, reverse gravure, dipping, and Langmuir Blodgett deposition.

The invention is not limited to the advantageous embodiment presented, but it may vary within the scope of the inventive idea presented in the claims.

## Claims

1. A method for measuring matter accumulated on the surface of skin or in hair, in which method

   - a measuring sensor (3) is placed in contact with the surface of the skin or hair, wherein matter from the surface of the skin or hair adheres to the surface of the measuring sensor (3),
   - the mass of the matter adhered to the measuring sensor (3) is weighed by determining the specific frequencies of the loaded measuring sensor (3) and the clean measuring sensor (3) and, by means of these, the change in the specific frequency of the measuring sensor (3) and in the mass, which is the mass of the matter adhered to the measuring sensor (3),
   **characterized in that**
   - the specific frequency of the measuring sensor (3) is determined by generating a frequency scanning signal on both sides of the specific frequency of the measuring sensor (3) and by guiding the frequency scanning signal through the measuring sensor (3),
   - the response of the frequency scanning signal in the measuring sensor (3) is measured and analyzed, thereby determining the specific frequency of the measuring sensor (3).

2. The method according to claim 1, **characterized in that** the measuring sensor (3) is used for weighing the secretion of sebum.

3. The method according to claim 1, **characterized in that** the measuring sensor (3) is used for weighing the cells peeling off and exfoliating from the surface of the skin, or remains of them.

4. The method according to claim 1, **characterized in that** the measuring sensor (3) is used for weighing water coming through the skin (transepidermal water loss, TEWL) or accumulated onto and evaporating from the surface of the skin.

5. The method according to claim 1, **characterized in that** the measuring sensor (3) is used for weighing cream dispensed onto the skin, or unabsorbed remains of it.

6. The method according to claim 1, **characterized in that** the measuring sensor (3) is used for weighing contaminants, impurities or other dirt accumulated onto the skin.

7. The method according to any of the claims 1 to 6, **characterized in that** the measuring sensor (3) used is a quartz crystal.

8. The method according to claim 7, **characterized in that** the measuring sensor (3) is cleaned and the same measuring sensor (3) is used again.

9. The method according to claim 7, **characterized in that** the measuring sensor (3) is replaced with a new measuring sensor (3) after a previous measurement.

10. The method according to any of the claims 1 to 9, **characterized in that** the measuring surface used for the measuring sensor (3) is a surface provided with a coating that is sensitive to the matter to be measured.

11. The method according to any of the claims 1 to 10, **characterized in that** the measuring surface used for the measuring sensor (3) is a surface provided with a pattern that is sensitive to the matter to be measured.

1

3

Fig. 1

2

3

4

4

4

5

8

8

6

7

9

Fig. 2

EP 2 375 248 A1

Fig. 3

Europäisches
Patentamt

European
Patent Office

Office européen
des brevets

## EUROPEAN SEARCH REPORT

Application Number

EP 11 39 7506

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/083279 A1 (3M INNOVATIVE PROPERTIES) 10 July 2008 (2008-07-10) * page 6, line 26 - page 7, line 24 * * page 12, line 10 - page 13, line 14 * ----- | 1-11 | INV. G01N29/02 A61B5/00 |
| A | US 2003/008407 A1 (FU) 9 January 2003 (2003-01-09) * paragraph [0117] * ----- | 1-11 | |
| Y | US 2010/087011 A1 (COOPER MATTHEW [GB]) 8 April 2010 (2010-04-08) * paragraphs [0110], [0149] * ----- | 1-11 | |
| | | | TECHNICAL FIELDS SEARCHED (IPC) |
| | | | G01N A61B |

The present search report has been drawn up for all claims

| Place of search | Date of completion of the search | Examiner |
|---|---|---|
| Munich | 17 August 2011 | Martelli, Luca |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 11 39 7506

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2011

| Patent document cited in search report | | Publication date | Patent family member(s) | | Publication date |
|---|---|---|---|---|---|
| WO 2008083279 | A1 | 10-07-2008 | EP<br>US | 2100130 A1<br>2010151553 A1 | 16-09-2009<br>17-06-2010 |
| US 2003008407 | A1 | 09-01-2003 | NONE | | |
| US 2010087011 | A1 | 08-04-2010 | EP<br>WO | 2122355 A1<br>2008114003 A1 | 25-11-2009<br>25-09-2008 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6966877 B
- US 4224950 A
- US 5935521 A
- US 5119828 A
- US 3863495 A